# EUROPÄISCHE PATENTSCHRIFT

(11) **EP 0 351 540 B1**
(45) Veröffentlichungstag und Bekanntmachung des Hinweises auf die Patenterteilung: **25.05.1994**
(21) Anmeldenummer: 89110440.8
(22) Anmeldetag: 09.06.1989
(51) Int. Cl.: A61B 17/32, A61B 17/28

(54) **Arthroskopie-Hakenstanze**
Arthroscopic hook-forceps
Pince à crochets d'arthroscopie

(30) Priorität: 22.07.1988 DE 3824910
(43) Veröffentlichungstag der Anmeldung: 24.01.1990
(73) Patentinhaber: Richard Wolf GmbH, 75438 Knittlingen (DE)
(72) Erfinder: Falk, Ernst, D-7137 Sternenfels-Diefenbach (DE)
(74) Vertreter: Wilcken, Hugo

(56) Entgegenhaltungen:
- EP-A- 0 119 405
- DE-A- 3 526 822
- DE-U- 8 712 835
- GB-A- 2 140 735

## Beschreibung

Die Erfindung geht von einer Arthroskopie-Hakenstanze aus, die aus einem aus dem distalen Ende eines runden Schaftes gebildeten feststehenden Stanzmaulteil und einem beweglichen Hakenmaulteil besteht, das mittels einer durch einen Scherengriff axial bewegbaren Schubstange verschwenkbar ist, die neben einem parallelen Spül- bzw. Absaugkanal durch den Schaft verläuft. Eine derartige Hakenstanze geht aus der DE-A-35 26 822 hervor.

Die bekannten Hakenstanzen nach der vorerwähnten Art weisen den Nachteil auf, daß die Schubstange unmittelbar mit den abzusaugenden Gewebe- oder Knochenteilen oder der verschmutzten Spül- oder Absaugflüssigkeit in Berührung kommt, so daß dadurch eine Keimverschleppung sowie eine Behinderung der Absaugung mit den bekannten Folgen möglich ist.

Die Aufgabe der Erfindung besteht darin, eine Arthroskopie-Hakenstanze der einleitend angeführten Art so zu verbessern, daß Keimverschleppungen vermieden sind, eine ungehinderte Absaugung gewährleistet und hierbei der Durchmesser des Schaftes so klein wie möglich gehalten ist, die Schubstange aber so ausgebildet ist, daß mit ihr eine hohe Betätigungskraft auf das bewegliche Hakenmaulteil übertragen werden kann.

Die Aufgabe der Erfindung wird durch die Merkmale des Patentanspruchs 1 gelöst.

Durch die Querschnittsaufteilung des Schaftes wird einerseits ein nahezu kreisrunder Querschnitt des Absaugkanals geschaffen, durch den auch größere Knochen- oder Knorpelstücke ohne Gefahr des Sichfestsetzens abgeführt werden können, und andererseits die Schubstange über ihren gesamten Umfang geführt, so daß bei vergleichbarem Querschnitt wesentlich höhere Betätigungskräfte übertragen werden können im Vergleich zu einer Schubstange gleichen Querschnittes mit Klauenführung. Im übrigen ist die Schubstange über die gesamte Länge des Schaftes vom Absaugkananl getrennt, wodurch die Gefahr einer Keimverschleppung deutlich verringert ist.

Um die erfindungsgemäße Arthroskopie-Hakenstanze auch mit den überlicherweise für solche Instrumente in der Klinik oder in der Praxis vorhandenen Scherengriffen verwenden zu können, ist eine Ausbildung gemäß Anspruch 2 vorgesehen.

Die Erfindung ist nachstehend anhand eines in der anliegenden Zeichnung dargestellten Ausführungsbeispiels näher beschrieben. Es zeigen:

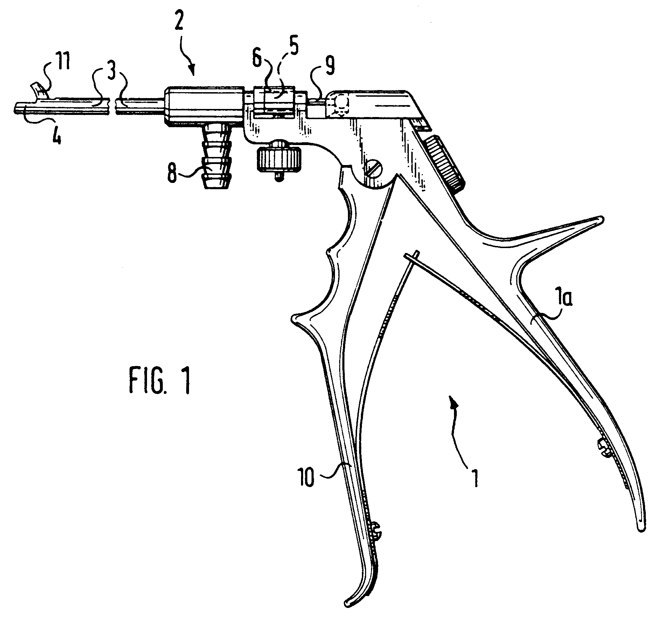

- Figur 1: eine Gesamtansicht der Arthroskopie-Hakenstanze,

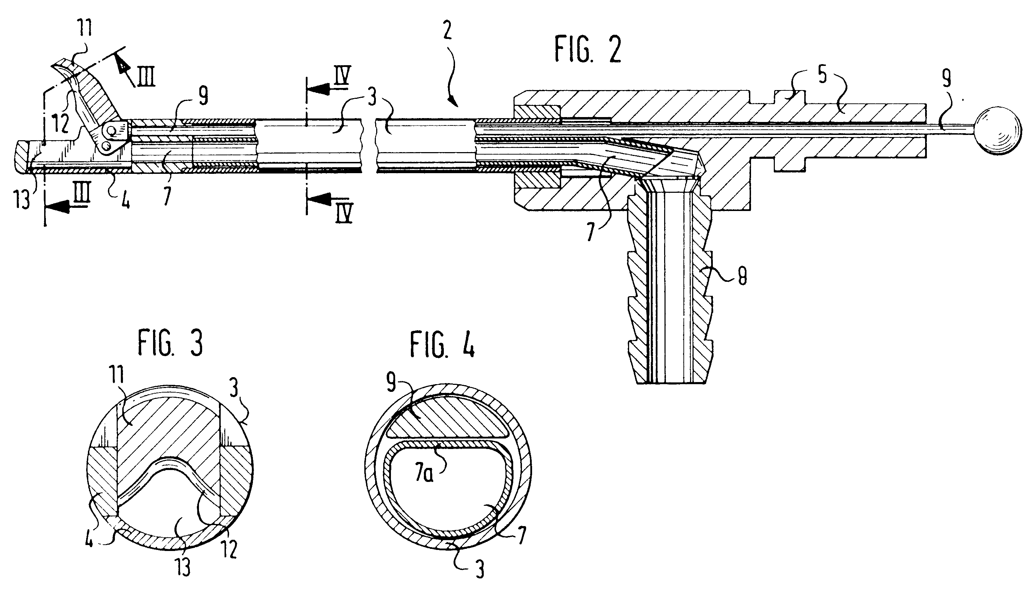

- Figur 2: die Hakenstanze ohne Handgriff und im wesentlichen im Längsschnitt,
- Figuren 3 und 4: zwei Querschnitte nach den Linien III-III und IV bis iV der Figur 2.

Die Arthroskopie-Hakenstanze besteht aus einem mit einem Scherengriff 1 zu kuppelnden Aufsatz 2 der Art, daß der Aufsatz 2 mit in einer Klinik oder einer Praxis bereits vorhandenen, bekannten Scherengriff verbindbar ist. Es können daher verschiedene, unterschiedliche Hakenstanzen für vorhandene Scherengriffe verwendet werden.

Die in den Größenabmessungen unterschiedlichen Aufsätze 2 bestehen jeweils aus einem Schaft 3, dessen distales Schaftende das feststehende Maulteil 4 bildet. Der Schaft 3 ist proximal mit einem Kupplungsteil 5 bekannter Art versehen, mit dem der feststehende Griffschenkel 1a eines Scherengriffs durch den Kupplungsteil 6 austauschbar verbindbar ist.

Durch den Schaft 3 verläuft ein Spül- bzw. Absaugkanal 7, der mit einem abgewinkelten, distal vor einem beweglichen Griffschenkel 10 liegenden Anschlußstutzen 8 für den Anschluß eines Unterdrucks versehen ist. Dieser Kanal 7 besitzt im wesentlichen Kreisquerschnitt, der oben mit einer Abflachung 7a versehen ist.

Diese Abflachung 7a und der obere Innenteil des Schaftes 3 bilden eine möglichst enge, abdichtende Führung für die dementsprechend profilierte Schubstange 9, die durch ihren Querschnitt die Übertragung großer Kräfte vom handbetätigten beweglichen Griffschenkel 10 auf das bewegliche Hakenmaulteil 11 zuläßt, wobei diese Ausführung zu einem geringen Druchmesser des Schaftes 3 bei verhältnismäßig weitem Absaugkanal 7 führt. Das Maulteil 11 ist mit einer unteren, erhältnismäßig tiefen Ausnehmung 12 versehen, die in der Lage ist, alle abgestanzten Gewebe- oder Knochenteile aufzunehmen. An die Ausnehmung 13 schließt sich bei geschlossenem Stanzmaul der Absaugkanal 7 direkt an.

Um eine möglichst hohe Absaugung im Schneidenbereich der beiden Maulteile zu erreichen, greifen das distale Ende der Schubstange 9 und das proximale Ende des beweglichen Maulteiles 11 im Bereich ihrer gelenkigen Verbindung formschlüssig ineinander, so daß das Absaugen der Spülflüssigkeit in diesem Bereich wirksam verhindert wird.

## Patentansprüche

1. Arthroskopie-Hakenstanze mit einem aus dem distalen Ende eines mit Kreisquerschnitt versehenen Schaftes (3) gebildeten feststehenden Stanzmaulteil (4) und einem beweglichen Hakenmaulteil, das mittels einer durch einen Scherengriff (1a, 10) axial bewegbaren Schubstange (9) verschwenbar ist, die neben einem Spül- bzw. Absaugkanal (7) durch den Schaft verläuft, dadurch gekennzeichnet, daß der sich an eine untere, tiefe Ausnehmung (12) des beweglichen Hakenmaulteiles (11) anschließende, durch den Schaft (3) verlaufende Absaugkanal (7) im wesentlichen einen kreisrunden, oben abgeflachten Querschnitt aufweist, dessen Abflachung (7a) und die obere Innenwand des Schaftes (3) die Führung für die Schubstange (9) bilden, deren Querschnitt dem Querschnitt der Führung angepaßt ist.

2. Arthroskopie-Hakenstanze nach Anspruch 1, dadurch gekennzeichnet, daß der Schaft (3), der den Absaugkanal (7) und dessen proximalen, seitlich abgewinkelten Anschlußstutzen (8) aufweist, durch eine Kupplung (5, 6) mit dem einen Griffschenkel (1a) des Scherengriffs lösbar verbunden ist und daß der andere Griffschenkel (10) des Scherengriffes mit dem proximalen Ende der mit dem beweglichen Hakenmaulteil (11) gelenkig verbundenen Schubstange (9) lösbar verbunden ist.

## Claims

1. Arthroscopy hook forceps having a fixed forceps mouth part (4) formed from the distal end of a shaft (3) provided with circular cross-section and a movable hook mouth part, which can be pivoted by means of an axially movable push rod (9) using a scissor grip (1a, 10), which push rod (9) extends through the shaft in addition to a rinsing or suction channel (7), characterised in that the suction channel (7) extending through the shaft (3) and connecting to a lower, deep recess (12) of the movable hook mouth part (11) has an essentially circular cross-section with a flattened portion at the top, the flattened portion (7a) of which and the upper inner wall of the shaft (3) forming the guide for tile push rod (9), the cross-section of which is adapted to the cross-section of the guide.

2. Arthroscopy hook forceps according to claim 1, characterised in that the shaft (3), which has the suction channel (7) and the proximal, laterally angled connection piece (8) thereof, is releasably connected to one gripping limb (1a) of the scissor grip by means of a coupling (5, 6), and in that the other gripping limb (10) of the scissor grip is releasably connected to the proximal end of the push rod (9) hingedly connected to the movable hook mouth part (11).

## Revendications

1. Pince à crochets pour arthroscopie, qui est constituée d'une partie de bouche formant pince (4) fixe formée de l'extrémité distale d'une tige (3) à section transversale circulaire et d'une partie de bouche à crochet mobile, qui peut pivoter à l'aide d'un bielle (9) déplaçable axialement par une poignée de cisaille (1a, 10), cette bielle s'étendant à travers la tige à côté d'un canal de lavage ou d'aspiration parallèle (7), caractérisée en ce que le canal d'aspiration (7) qui se raccorde à un évidement inférieur profond (12) de la partie de bouche à crochet mobile (11) et qui s'étend à travers la tige (3) présente en substance une section transversale circulaire, aplatie dans la partie supérieure, dont le méplat (7a) forme avec la paroi interne supérieure de la tige (3) la coulisse de la bielle (9) dont la section transversale est adaptée à la section transversale de la coulisse.

2. Tige à crochet pour arthroscopie selon la revendication 1, caractérisée en ce que la tige (3), qui comporte le canal d'aspiration (7) et son tube de raccordement proximal latéralement coudé (8), est raccordée par un accouplement (5, 6) de manière amovible à la première branche (1a) de la poignée de cisaille et l'autre branche (10) de la poignée de cisaille est raccordée à l'extrémité proximale de la bielle (9) articulée sur la partie de bouche à crochet mobile (11).
